Europäisches Patentamt

European Patent Office

Office européen des brevets

(11) Veröffentlichungsnummer: **0 338 410 B1**

# EUROPÄISCHE PATENTSCHRIFT

(45) Veröffentlichungstag der Patentschrift: **28.09.94**

(51) Int. Cl.5: **C12R 1/19**, C12N 1/20, C12N 15/70

(21) Anmeldenummer: **89106554.2**

(22) Anmeldetag: **13.04.89**

(54) **Sekretormutante von Escherichia coli.**

(30) Priorität: **19.04.88 DE 3813107**

(43) Veröffentlichungstag der Anmeldung:
**25.10.89 Patentblatt 89/43**

(45) Bekanntmachung des Hinweises auf die
Patenterteilung:
**28.09.94 Patentblatt 94/39**

(84) Benannte Vertragsstaaten:
**AT BE CH DE FR GB LI NL SE**

(56) Entgegenhaltungen:
**EP-A- 0 071 485**
**WO-A-88/05821**

**PATENT ABSTRACTS OF JAPAN, Band 12,
Nr. 11 (C-468)[2858], 13. Januar 1988 & JP-
A-62166881**

**JOURNAL OF BACTERIOLOGY, Band 145, Nr.
3, März 1981, Seiten 1351-1358;
J.-C.LAZZARONI et al."Genetic and biochemical characterization of periplasmic-leaky
mutants of Escherichia coli K-12"**

(73) Patentinhaber: **Consortium für elektrochemische Industrie GmbH
Zielstattstrasse 20
D-81379 München (DE)**

(72) Erfinder: **Böck, August, Prof. Dr. rer. nat.
Lindenstrasse 10
D-8085 Geltendorf (DE)**
Erfinder: **Seydel, Wiebke, Dipl.-Biol.
Römerbergstrasse 10
D-7951 Warthausen-Birkenhart (DE)**
Erfinder: **Schmid, Gerhard, Dr. Dipl.-Chem.
Dorfstrasse 9a
D-8000 München 60 (DE)**
Erfinder: **Müller, Gabriele, Dipl.-Biol.
Wendelsteinstrasse 7
D-8011 Aschheim (DE)**

PROCEEDINGS OF THE NATIONAL ACADEMY OF SCIENCES OF USA, Band 74, Nr. 4, April1977, Seiten 1417-1420, Washington, US; Y. HIROTA et al.: "On the process of cellular division in Escherichia coli: A mutant of E. coli lacking a murein-lipoprotein"

JOURNAL OF BACTERIOLOGY, Band 133, Nr. 3, März 1978, Seiten 1419-1426; D.W. YEM et al.:"Physiological characterization of an Escherichia coli mutant altered in the structure of murein lipoprotein"

**Beschreibung**

Die Erfindung betrifft Escherichia-coli-Stämme zur massiven Proteinsekretion in das Kulturmedium, Verfahren zu ihrer Herstellung und ihre Verwendung.

Aufgrund der Zellwandstruktur von gramnegativen Bakterien ging man bisher davon aus, daß eine wirkliche Sekretion von Proteinen in das Kulturmedium nicht stattfinden kann, da die äußere Membran eine starke Permeabilitätsbarriere darstellt. Natürlicherweise ist das Bakterium E. coli nicht in der Lage, Proteine zu sekretieren bzw. in das Kulturmedium auszuschleusen. Nur Proteinexport in das Periplasma ist bei E. coli möglich. Zwei Ausnahmen bilden die Proteine Haemolysin und Colicine. Diese Exoproteine stellen aber Sonderfälle dar, da für das jeweilige Exoprotein spezifische Sekretionsproteine am Ausschleusungsprozeß beteiligt sind; vgl. Wagner et al., J. Biol. Chem., 369 (1988) 39-46.

Ferner sind in der Literatur artifizielle Sekretions-Systeme für E. coli beschrieben, die jedoch nur zu geringen Exoproteinausbeuten im Kulturmedium führen oder in ihrer Anwendbarkeit beschränkt sind.

(a) Sogenannte "leaky"-Mutanten. Derartige Mutanten sind beispielsweise von E. coli K12 bekannt, wobei aufgrund eines Defekts in der äußeren Membran periplasmatische bzw. periplasmatisch angereicherte Proteine in das Kulturmedium gelangen. Es handelt sich um einen unspezifischen Mechanismus; vgl. Lazzorni & Portalier, J. Bact., 145 (1981) 1351-1358. Bei diesen "leaky"-Mutanten handelt es sich zum Teil um Stämme mit veränderter Lipopolysaccharidstruktur, zum Teil um Stämme mit veränderten Lipoproteinanteilen in der äußeren Membran; vgl. Hirota et al., Proc. Natl. Acad. Sci. USA, 74 (1977) 1417-1420, sowie Yem & Wu, J. Bacteriol., 133 (1978) 1419-1426. Auch Mutationen in den sogenannten "tol"-loci führen über Aktivierung der Phospholipase A zum "leaky"-Phänotyp. Es kann sich dabei um spezifisches oder unspezifisches "leakage" handeln.

(b) Protein-Sekretion durch Protein A-Fusion gemäß EP-A-0 225 860 = 86.850 415.0. Grundlage dieses Stands der Technik ist die Beobachtung, daß die Induktion von filamentösem Wachstum in E. Coli zur Sekretion von periplasmatischen Proteinen in das Kulturmedium führt. E. coli zeigt filamentöses Wachstum bei Induktion der sogenannten Hitze-Schock-Antwort. Wenn also die Expression des gewünschten Genprodukts abhängig gemacht wird von der Hitze-Schock-Antwort, dann kann eine Sekretion des gewünschten, im Periplasma angereicherten Proteins erzielt werden. In diesem Verfahren macht man sich zunutze, daß Protein A aus Staphylococcus aureus sowie Fragmente dieses Proteins in E. coli eine Hitze-Schock-Antwort auslösen. Wahrscheinlich stellt das Protein A in E. coli sogar selbst ein Hitze-Schock-Protein dar, da es stromaufwärts von eigentlichen Promotor eine sigma-32-artige Promotorstruktur besitzt, wodurch sich der Effekt noch potenziert. Verknüpft man also Promotorsequenz, Signalsequenz und N-terminale Fragmente von protein A mit der genetischen Information des gewünschten Proteins, so kann über die Hitze-Schock-Antwort und filamentöses Wachstum eine Sekretion periplasmatischer Proteine in das Kulturmedium erfolgen. Dieses System ist allerdings insofern problematisch, als durch die Hitze-Schock-Antwort zusätzlich die ATP-abhängige Protease La induziert wird. Es kommt daher zum proteolytischen Abbau des gewünschten Proteins. Daher wäre es erwünscht, einen La-defizienten Stamm (lon⁻; vgl. Young & Davis, Proc. Natl. Acad. Sci. USA, 80 (1983) 1194-1198) für die Produktion zu verwenden. Ein derartiger Stamm wurde jedoch bisher nicht bereitgestellt.

(c) Extrazelluläre Herstellung von Penicillinase und Xylanase gemäß EP-A-0 121 138 = 84 102 440.9. Dieser Stand der Technik betrifft ein Verfahren unter Verwendung spezieller E.-coli-Konstrukte zur Sekretion von Penicillinase und Xylanase in das Kulturmedium. Dieses bekannte Verfahren ist also auf die Gewinnung spezieller Proteine beschränkt.

(d) Patent Abstracts of Japan Bd. 12, Nr. 11 (C 468) [2858] vom 13. Januar 1988 beschreibt einen E. coli Stamm, der durch Mutation des Stammes E. coli HJ-2 mit N-methyl-N'-nitro-N-nitrosoguanidin erhalten wurde. Dieser Stamm kann temperaturkontrolliert periplasmatische Proteine wie beispielsweise β-Lactamase aus der Zelle sekretieren.

Der vorliegenden Erfindung liegt nun die Aufgabe zugrunde, E. coli-Stämme zur Proteinsekretion von mindestens 140 mg Protein/l innerhalb von 48 h in das Kulturmedium sowie Verfahren zu ihrer Herstellung und Verwendung bereitzustellen. Teilaufgaben sind

- eine Exoprotein-Produktion in E. coli als einem nicht pathogenen Organismus
- eine möglichst hohe Sekretionsrate und
- eine einfache Aufarbeitung des Exoproteins aus der Kultur (ggf. nach Filtration).

Die vorstehend genannte Aufgabe wird gemäß einer Ausführungsform der Erfindung durch ein Verfahren gelöst, welches dadurch gekennzeichnet ist, daß man

(a) in an sich bekannter Weise das Strukturgen eines zu exprimierenden Exoproteins in ein für die Expression geeignetes Plasmid zu einem Hybridplasmid integriert,

(b) in an sich bekannter Weise einen E.-coli-Stamm mit minA- und/oder minB-Mutation oder einen E.-coli-Stamm mit einer Mutation in einem Protein oder in mehreren Proteinen der äußeren Membran mit dem gebildeten Hybridplasmid transformiert,

(c) in an sich bekannter Weise den transformierten E.-coli-Stamm einer Mutagenese unterwirft und

(d) gegebenenfalls danach zellwandaktiven Substanzen aussetzt,

(e) in an sich bekannter Weise das den Stufen (c) und ggf. (d) unterworfene Zellmaterial einem Screening auf E.-coli-Mutanten mit gegenüber dem eingesetzten E.-coli-Stamm erhöhter Proteinsekretion unterwirft und

(f) gegebenenfalls in an sich bekannter Weise die gemäß Stufe (e) gewonnene E.-coli-Mutante(n) mit erhöhter Proteinsekretion plasmidfrei macht.

Unter dem Begriff des Strukturgens für ein Exoprotein wird hier (Stufe a) und im folgenden die das gewünschte Protein kodierende DNA-Sequenz einschließlich ihrer oder einer für den Export essentiellen Signalsequenz (Leadersequenz) verstanden.

Die Mutagenese kann chemisch durchgeführt werden, beispielsweise mit N-Methyl-N'-nitro-N-nitrosoguanidin (MNNG).

Ein Beispiel für eine zellwandaktive Substanz ist D-Cycloserin.

Insbesondere geht man beim erfindungsgemäßen Verfahren von E. coli DS 410 beziehungsweise E. coli BW 7261 aus.

Gemäß dem erfindungsgemäßen Verfahren kann man den Ausgangsstamm mit einem Hybridplasmid transformieren, das das Strukturgen des Proteins umfaßt, dessen Produktion erwünscht ist.

Man kann das erfindungsgemäße Verfahren jedoch auch so durchführen, daß man den Ausgangsstamm zuerst mit einem Hybridplasmid transformiert, das das Strukturgen eines Indikatorproteins umfaßt, mit dem sich auf einfache Weise Mutanten screenen lassen, die die gewünschte Eigenschaft einer massiven Proteinsekretion in das Kulturmedium besitzen. Ist eine derartige Mutante mit Hilfe des Indikatorproteins ermittelt, so wird diese Mutante gemäß der hier angesprochenen Variante des erfindungsgemäßen Verfahrens plasmidfrei gemacht, wonach man die plasmidfreie Mutante mit einem anderen Hybridplasmid transformiert, das das Strukturgen eines Proteins umfaßt, dessen technische Produktion erwünscht ist.

Ein Beispiel für ein Indikatorprotein ist CGT, da seine Fähigkeit zur Polysaccharid-Hydrolyse (Stärke-Hydrolyse) als Indikatorreaktion ein bequemes Screenen erlaubt. Bei der Verwendung von CGT wird das für das Exoprotein kodierende DNA-Fragment zusammen mit der eigenen, für den Export essentiellen Signalsequenz unter die Kontrolle eines starken Promotors gestellt, der einfach und gezielt in seiner Aktivität reguliert werden kann, beispielsweise unter die Kontrolle des tac-Promotors. Dazu kann der genannte DNA-Abschnitt in das "multicopy-number"-Plasmid pJF118ut integriert werden. Nach Transformation eines E.-coli-Stammes mit minA- und/oder min-B-Mutation (Minizell-Stamm), wie E. coli DS 410, beziehungsweise mit einer Mutation in einem Protein oder in mehreren Proteinen der äußeren Membran, wie E. coli BW 7261, mit dem rekombinanten Plasmid wird der Rezipient einer chemischen Mutagenese unterworfen und anschließend membran- bzw. zellwandaktiven Substanzen ausgesetzt. Eine Resistenz gegen D-Cycloserin ist erfahrungsgemäß ein brauchbares Indiz für eine verbesserte Ausschleusung. Die Behandlung mit der zellwandaktiven Substanz wird zweckmäßigerweise mehrfach wiederholt. Potentielle Sekretormutanten werden aus der Vielzahl der erhaltenen Mutanten mit einem einfachen Screeningsystem ausgewählt. Die quantitative Bewertung der Mutanten erfolgt durch einen Enzymtest oder Immunoblotting.

Im übrigen kann sich der Fachmann bezüglich der Hybridplasmid-Bildung, der Transformation, der Mutagenese, der Behandlung mit zellwandaktiven Substanzen, dem Screenen, der Plasmid-Entfernung, der Kultivierung der Bakterien sowie der Proteingewinnung an den einschlägigen Stand der Technik halten, etwa an die eingangs zitierte Literatur.

Die vorstehend genannte Aufgabe wird weiter gelöst durch einen E. coli Stamm mit einer minA- und/oder minB-Mutation oder einer Mutation in einem Protein oder in mehreren Proteinen der äußeren Membran zur Proteinsekretion von mindestens 140 mg Protein/l innerhalb 48 Stunden in das Kulturmedium, wie er nach dem bereits beschriebenen Verfahren herstellbar ist.

Die Erfindung bietet folgende Vorteile:

(a) Die erfindungsgemäßen E.-coli-Stämme produzieren Exoproteine in industriell interessantem Maßstab.

(b) Das von den erfindungsgemäßen Mutanten hergestellte Exoprotein kann kostengünstig und mit geringem Zeitaufwand aufgereinigt bzw. direkt weiter verwendet werden.

Während bekannte Bakterien, die Exoproteine überproduzieren, ihre Produkte zu "Inclusionbodies" (Einschlußkörpern) aggregiert intrazellulär ablagern, schleusen die erfindungsgemäßen Mutanten überproduziertes Exoprotein quantitativ in das Kulturmedium aus. Damit entfallen die zeitaufwendigen und zum Teil komplizierten Prozesse des Zellaufbruchs und der Renaturierung der "Inclusionbodies". "Inclusionbodies"

4

bestehen oftmals aus den Prä-Formen des zu exportierenden Proteins, besitzen also noch die Signalsequenz und zeigen somit keine vollständige katalytische Funktion. Da die Renaturierung in wenigen Fällen in zufriedenstellendem Ausmaß gelingt, stellt die Exoprotein-Produktion mit Hilfe der erfindungsgemäßen Mutanten eine große Verfahrenserleichterung dar. Das Exoprotein muß nur aus der Kultur (ggf. nach Filtration) aufgereinigt werden oder kann aufgrund der hohen Ausbeuten im Fall von Exoenzymen direkt in die gewünschte Enzymreaktion eingesetzt werden.

Die erfindungsgemäßen Mutanten können im Bereich der bakteriellen Exoprotein-Produktion breite Anwendung finden. Ein gewünschtes rekombinantes Plasmid, das die genetische Information für ein kommerziell interessantes Protein trägt, kann in den erfindungsgemäßen Mutanten mit Hilfe der Methoden der rekombinanten DNA-Technik eingebracht werden. Im Laufe der stationären Phase des bakteriellen Wachstums wird das Exoprotein durch die erfindungsgemäße Mutante spezifisch in das Kulturmedium sezerniert. Damit ist eine einfache, schnelle und kostengünstige Aufreinigung des gewünschten Proteins möglich. Handelt es sich bei dem Exoprotein um ein Enzym, so kann der Kulturüberstand aufgrund der hohen Enzymausbeuten von beispielsweise 250 mg/l oder mehr direkt in die anschließende Enzymreaktion eingesetzt werden.

Nachstehend wird die Erfindung durch Beispiele näher erläutert.

Beispiel 1 (Herstellung einer erfindungsgemäßen E.-coli-Mutante)

Zur Herstellung einer Sekretormutante wählte man das Plasmid pCM703, das die genetische Information für eine CGT enthält, und E. coli DS 410. CGT wurde als Indikator für Mutanten mit verbesserter Enzymsekretion gewählt. Dementsprechend wurde E. coli DS 410 (pCM703) in Flüssigkultur in Vollmedium bei 37 °C angezogen. Nach dem Erreichen einer optischen Dichte von 1 wurden der Kultursuspension 20 $\mu$g/ml N-Methyl-N'-nitro-N-nitrosoguanidin (MNNG) zugesetzt, wonach weitere 30 min lang bei 37 °C inkubiert wurde (etwa 90 % Abtötung). Durch Waschen der Zellen mit Phosphatpuffer (pH 7.0) wurde die Wirkung des Mutagens unterbrochen. Aliquots der Zellsuspension wurden auf LB-Platten (2 % Stärke, 0,5 % Lactose und steigende Konzentrationen an D-Cycloserin) ausplattiert. Die für die Selektion eingesetzten Antibiotikum-Konzentrationen betrugen 200 bis 1 000 $\mu$g D-Cycloserin/ml, wobei die Konzentration nach jedem Mutageneseschritt verdoppelt wurde. Nach 48 h Bebrütung bei 37 °C erfolgte die Auswahl der Mutanten aufgrund der Größe des Stärkehydrolysehofes, der auf die Aktivität der sezernierten CGT zurückzuführen ist. Potentiell verbesserte Ausschleusung des plasmidkodierten Enzyms wurde durch Ausstreichen auf Indikatorplatten (0,5% Amylopektinazur) überprüft und in einem enzymatischen Test bestätigt. Aus einer Vielzahl von Mutanten wurde der E.-coli-Stamm ausgewählt, der die höchste extrazelluläre Enzymaktivität aufwies (bezeichnet: E. coli DS410 K5). Durch mehrmalige Anzucht in antibiotikumfreiem Medium konnte die Mutante plasmidfrei gemacht werden.

Beispiel 2 (CGT-Sekretion)

Die erfindungsgemäße plasmidfreie Mutante E. coli DS410 K5 wurde mit dem Plasmid pCM703 (das für eine CGT kodiert) retransformiert. Der rekombinante Stamm wurde in LB-Flüssigmedium (0,5 % Laktose und 0,2 % Glukose) bei 30 °C angezogen. Nach 24 h, 48 h bzw. 72 h Inkubation wurde jeweils ein Aliquot der Zellsuspension entnommen. Danach wurden die Zellen abzentrifugiert und der verdünnte Kulturüberstand mit einem gleichen Volumen einer 10-proz. Stärkelösung bei 40 °C inkubiert. Nach 5 min Inkubationsdauer wurden 1,5 Volumenteile Methanol zugegeben, was zur Präzipitation nicht umgesetzter Stärke führte. Die gefällte Stärke wurde abzentrifugiert, wonach der Cyclodextringehalt im Überstand mit Hilfe einer HPLC-Anlage ermittelt wurde. Mann erhielt folgende Ergebnisse:

|  | Einheiten/ml | mg Protein/l |
| --- | --- | --- |
| 24 h | 16 – 20 | 64 – 80 |
| 48 h | 45 – 50 | 180 – 200 |
| 72 h | 55 – 60 | 220 – 240 |

Vergleichsbeispiel 1

Beispiel 2 wurde mit der Ausnahme wiederholt, daß das Plasmid pCM703 in E. coli HB101 transformiert wurde. Die Anzucht und die Bestimmung der extrazellulären Enzymaktivität erfolgten analog. Man erhielt folgende Ergebnisse:

|  | Einheiten/ml | mg Protein/l |
|---|---|---|
| 24 h | 0,5 | 2 |

Beispiel 3 (Sekretion von $\beta$-Lactamase)

Die erfindungsgemäße Mutante E. coli DS410 K5 wurde mit dem Plasmid pAP5 transformiert, das die genetische Information für $\beta$-Lactamase trägt. In diesem rekombinanten Plasmid steht das $\beta$-Lactamase-Gen unter der Kontrolle des tac-Promotors, der durch Induktion mit beispielsweise Lactose die Überexpression der $\beta$-Lactamase bewirkt. Die Anzucht von E. coli DS410 K5 (pAP5) und die Probennahme nach 48-stündiger Inkubation zur Bestimmung der extrazellulären $\beta$-Lactamase-Aktivität erfolgten gemäß Beispiel 2. Die extrazelluläre $\beta$-Lactamase-Aktivität wurde mit Hilfe eines chromogenen Substrats, nämlich Nitrocefin, spektralphotometrisch bei 480 nm bestimmt; vgl. Biochemistry, 23 (1984) 1282-1287. Man erhielt folgende Ergebnisse:

| Inkubationsdauer | Enzymaktivität im Überstand (relative Zahlen) |
|---|---|
| 48 h | 86 |

Vergleichsbeispiel 2

Beispiel 3 wurde mit der Abänderung nachgearbeitet, daß der rekombinante Stamm E. coli HB101 (pAP5) verwendet wurde. Man erhielt folgende Ergebnisse:

| Inkubationsdauer | Enzymaktivität im Überstand (relative Zahlen) |
|---|---|
| 48 h | 0,1 |

Beispiel 4

Die Vorgehensweise gemäß den Beispielen 1 und 2 wurde wiederholt mit der Abänderung, daß anstatt E. coli DS 410 der E. coli Stamm BW 7261 (ton A22, ompF 627) mit dem Plasmid pCM 703 verwendet wurde. Die daraus erhaltene Mutante wurde plasmidfrei gemacht und mit dem Plasmid pCM 703 retransformiert. Dieser Stamm wurde in flüssigem Vollmedium (LB), das 0,5 % Laktose und 0,2 % Glukose enthält, bei 30 °C angezogen. Nach 48 h bzw. 72 h Inkubation wurde die Enzymaktivität im Überstand bestimmt (siehe Beispiel 2).

|  | Einheiten/ml | mg Protein/l |
|---|---|---|
| 48 h | 28 | 140 |
| 72 h | 42 | 210 |

**GLOSSAR**

E.-coli-Stamm mit minAB-Mutation: Davie et al., Bacteriol., 158 (1984) 1202 - 1203.

E. coli DS410: DSM 4513, Deutsche Sammlung von Mikroorganismen, Mascheroder Weg 1b, 3300 Braunschweig.

E.-coli-Stamm mit einer Mutation von Protein in der äußeren Membran: Wanner, J. Mol. Biol. 191 (1986) 39.

E. coli BW 7261, DSM 5231, Deutsche Sammlung von Mikroorganismen, Mascheroder Weg 1b, 3300 Braunschweig.

E. coli HB101: z.B. DSM 1607

| | |
|---|---|
| pJF118ut: | Fig. 1 |
| pCM703: | Fig. 2 |
| pAP5: | Fig. 3 |
| tac: | tac-Promotor; vgl. De Boer et al., Proc. Natl. Acad. Sci. USA, 80 (1983) 21-25 |
| CGT: | Strukturgen für CGT (Cyclodextringlycosyltransferase) (einschließlich Signalsequenz) |
| t: | Terminator |
| amp: | Ampicillin-Resistenz |
| tet: | Tetracyclin-Resistenz |
| lacI: | lac-Repressor |

Liste angeführter Restriktionsenzyme:

BamH I
Cla I
EcoR I
Hind III
Nru I
Pst I
Pvu I
Sca I

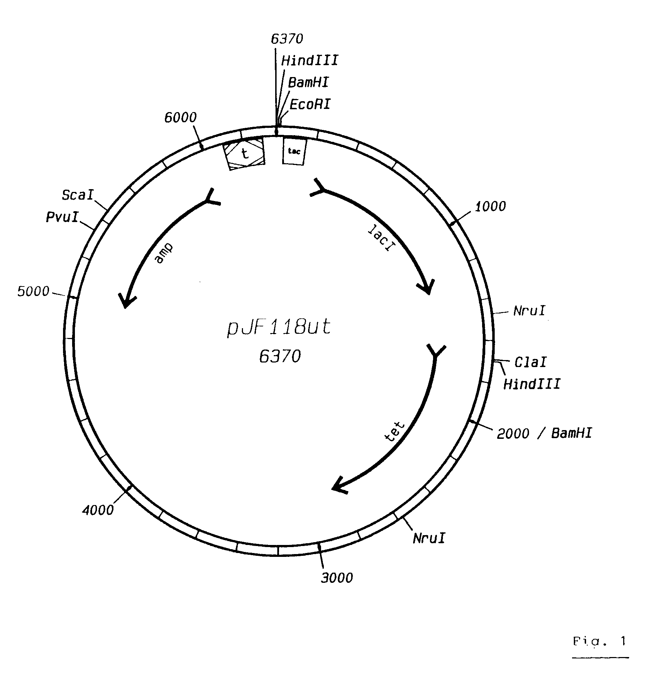

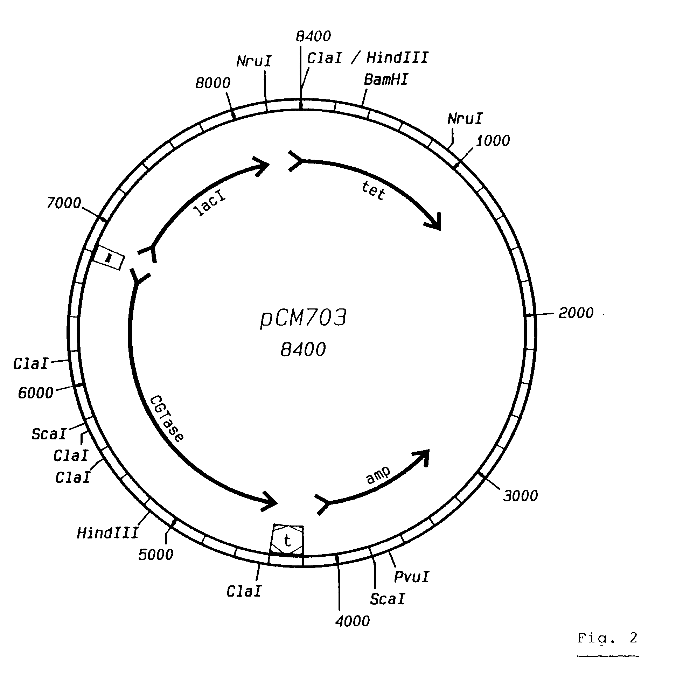

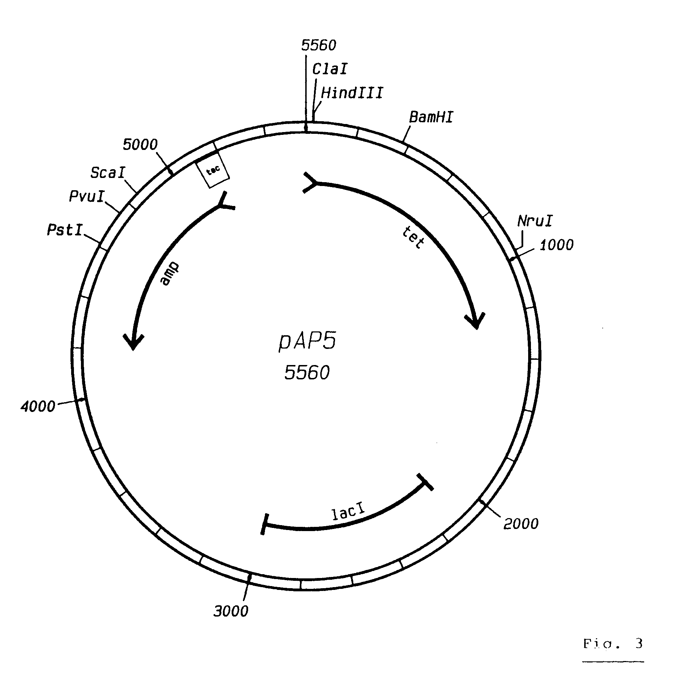

**Patentansprüche**

1. Verfahren zur Herstellung eines E.-coli-Stammes zur Proteinsekretion von mindestens 140 mg Protein/l innerhalb von 48 Stunden in das Kulturmedium, dadurch gekennzeichnet, daß man

   (a) in an sich bekannter Weise das Strukturgen eines zu exprimierenden Exoproteins in ein für die Expression geeignetes Plasmid zu einem Hybridplasmid integriert,

   (b) in an sich bekannter Weise einen E.-coli-Stamm mit minA- und/oder minB-Mutation oder einen E.-coli-Stamm mit einer Mutation in einem Protein oder in mehreren Proteinen der äußeren Membran mit dem gebildeten Hybridplasmid transformiert,

   (c) in an sich bekannter Weise den transformierten E.-coli-Stamm einer Mutagenese unterwirft und

   (d) gegebenenfalls danach zellwandaktiven Substanzen aussetzt,

   (e) in an sich bekannter Weise das den Stufen (c) und ggf. (d) unterworfene Zellmaterial einem Screening auf E.-coli-Mutanten mit gegenüber dem eingesetzten E.-coli-Stamm erhöhter Proteinsekretion unterwirft und

   (f) gegebenenfalls in an sich bekannter Weise die gemäß Stufe (e) gewonnene E.-coli-Mutante(n) mit erhöhter Proteinsekretion plasmidfrei macht.

2. Verfahren nach Anspruch 1, dadurch gekennzeichnet, daß man in Stufe (c) gemäß Anspruch 1 chemisch mutagenisiert, beispielsweise mit N-Methyl-N'-nitro-N-nitrosoguanidin (MNNG).

7

3. Verfahren nach Anspruch 1 oder 2, dadurch gekennzeichnet, daß man in Stufe (d) gemäß Anspruch 1 als zellwandaktive Substanz D-Cycloserin verwendet.

4. Verfahren nach Anspruch 1, dadurch gekennzeichnet, daß man E. coli DS 410 (DSM 4513) einsetzt.

5. Verfahren nach Anspruch 1, dadurch gekennzeichnet, daß man E. coli BW 7261 (DSM 5231) einsetzt.

6. Verfahren nach einem der Ansprüche 1 bis 5, dadurch gekennzeichnet, daß man in Stufe (a) gemäß Anspruch 1 das CGT-Strukturgen verwendet, in Stufe (e) gemäß Anspruch 1 eine Polysaccharid-Hydrolyse des gebildeten CGT als Indikatorreaktion für E.-coli-Mutanten mit erhöhter Proteinsekretion wählt und Stufe (f) gemäß Anspruch 1 folgen läßt.

7. E. coli Stamm mit einer minA- und/oder minB-Mutation oder einer Mutation in einem Protein oder in mehreren Proteinen der äußeren Membran zur Proteinsekretion von mindestens 140 mg Protein/l innerhalb 48 Stunden in das Kulturmedium, herstellbar gemäß einem oder mehreren der Ansprüche 1-6.

8. Verwendung eines E.-coli-Stammes gemäß Anspruch 7 zur Produktion eines gewünschten Proteins durch massive Sekretion in das Kulturmedium.

## Claims

1. Process for the preparation of an E. coli strain for protein secretion of at least 140 mg of protein/l within 48 hours into the culture medium, characterized in that
   (a) the structural gene of an exoprotein which is to be expressed is integrated, in a manner known per se, into a plasmid which is suitable for expression, to give a hybrid plasmid,
   (b) an E. coli strain with a minA and/or minB mutation or an E. coli strain with a mutation in one protein or in several proteins of the outer membrane is transformed, in a manner known per se, with the hybrid plasmid which has been formed,
   (c) the transformed E. coli strain is subjected, in a manner known per se, to mutagenesis, and
   (d) where appropriate is subsequently exposed to substances acting on the cell wall,
   (e) the cell material subjected to stages (c) and, where appropriate, (d) is subjected, in a manner known per se, to a screening for E. coli mutants with increased protein secretion compared with the E. coli strain used and
   (f) where appropriate the E. coli mutant(s) with increased protein secretion obtained as in stage (e) is (are) rendered plasmid-free, in a manner known per se.

2. Process according to Claim 1, characterized in that chemical mutagenesis is carred out in stage (c) in Claim 1, for example with N-methyl-N'-nitro-N-nitrosoguanidine (MNNG).

3. Process according to Claim 1 or 2, characterized in that D-cycloserine is used as substance acting on the cell wall in stage (d) in Claim 1.

4. Process according to Claim 1, characterized in that E. coli DS 410 (DSM 4513) is used.

5. Process according to Claim 1, characterized in that E. coli BW 7261 (DSM 5231) is used.

6. Process according to any of Claims 1 to 5, characterized in that the CGT structural gene is used in stage (a) in Claim 1, a polysaccharide hydrolysis of the CGT formed is chosen as indicator reaction for E. coli mutants with increased protein secretion in stage (e) in Claim 1, and stage (f) as in Claim 1 follows.

7. E. coli strain with a minA and/or minB mutation or a mutation in one protein or in several proteins of the outer membrane for protein secretion of at least 140 mg of protein/l within 48 hours into the culture medium, which can be prepared according to one or more of Claims 1-6.

8. Use of an E. coli strain according to Claim 7 for the production of a desired protein by massive secretion into the culture medium.

EP 0 338 410 B1

**Revendications**

1.  Procédé de préparation d'une souche de E. coli pour la sécrétion de protéines dans le milieu de culture à raison d'au moins 140 mg de protéine par litre en 48 heures, procédé caractérisé en ce que :
    (a) on intègre de manière en elle-même connue le gène de structure d'une exoprotéine à exprimer dans un plasmide propre à l'expression, pour former un plasmide hybride,
    (b) on transforme de manière connue avec ce plasmide hybride une souche de E. coli à mutation minA et/ou minB ou une souche de E. coli avec une mutation dans une ou plusieurs protéines de la membrane extérieure,
    (c) on soumet de manière connue la souche transformée à une mutagenèse, et
    (d) le cas échéant on l'expose ensuite à des substances actives à l'égard de la paroi cellulaire,
    (e) on soumet de manière connue la matière cellulaire ayant subi l'opération (c) et éventuellement l'opération (d) à un screening sur mutants de E. coli à sécrétion protéique accrue par rapport à la souche employée, et
    (f) le cas échéant on élimine le plasmide du ou des mutant(s) obtenu(s) dans l'opération (e), à sécrétion protéique accrue.

2.  Procédé selon la revendication 1, caractérisé en ce que dans l'opération (c) on effectue la mutagenèse par voie chimique, par exemple avec la N-méthyl-N'-nitro-N-nitrosoguanidine (MNNG).

3.  Procédé selon la revendication 1 ou 2, caractérisé en ce que dans l'opération (d) on utilise de la D-cyclosérine comme substance active à l'égard de la paroi cellulaire.

4.  Procédé selon la revendication 1, caractérisé en ce que l'on utilise la souche E. coli DS410 (DSM 4513).

5.  Procédé selon la revendication 1, caractérisé en ce que l'on utilise la souche E. coli BW7261 (DSM 5231).

6.  Procédé selon l'une des revendications 1 à 5, caractérisé en ce que dans l'opération (a) on utilise le gène de structure de la CGT, dans l'opération (e) on prend une hydrolyse de polyoses de la CGT formée comme réaction indicatrice pour mutants de E. coli à sécrétion protéique accrue, et on fait suivre ensuite l'opération (f).

7.  Souche de E. coli avec une mutation minA et/ou minB ou une mutation dans une ou plusieurs protéines de la membrane extérieure, pour la sécrétion de protéines dans le milieu de culture à raison d'au moins 140 mg de protéine par litre en 48 heures, souche que l'on peut obtenir selon une ou plusieurs des revendications 1 à 6.

8.  L'emploi d'une souche de E. coli selon la revendication 7 pour la production d'une protéine voulue par sécrétion massive dans le milieu de culture.

9

Fig. 1

pCM703
8400

NruI 1000
2000
3000
BamHI
ClaI / HindIII
8400
NruI
8000
7000
tet
lacI
amp
CGTase
PvuI
ScaI
4000
ClaI
HindIII
5000
ScaI
ClaI
ClaI
6000
ClaI

Fig. 2

Fig. 3